# EUROPEAN PATENT SPECIFICATION

(11) **EP 3 032 236 B1**
(45) Date of publication and mention of the grant of the patent: **21.03.2018**
(21) Application number: 14196957.6
(22) Date of filing: 09.12.2014
(51) Int. Cl.: G01M 3/28, E03B 7/00, G01P 13/00, G01F 1/38, G01F 1/56, B01D 35/153, A61M 39/24, F16K 15/00, A61M 5/14, F17D 3/01

(54) **FLUID FLOW DETECTION DEVICE AND ASSEMBLY COMPRISING A FLUID FLOW DETECTION DEVICE**
FLÜSSIGKEITSSTRÖMUNGSDETEKTIONSVORRICHTUNG UND ANORDNUNG MIT EINER FLÜSSIGKEITSSTRÖMUNGSDETEKTIONSVORRICHTUNG
DISPOSITIF DE DÉTECTION D'ÉCOULEMENT DE FLUIDE ET ENSEMBLE COMPRENANT UN DISPOSITIF DE DÉTECTION D'ÉCOULEMENT DE FLUIDE

(43) Date of publication of application: 15.06.2016
(73) Proprietor: Honeywell Technologies Sarl, 1180 Rolle (CH)
(72) Inventor: KUSALA, Petr, 68201 Vyskov (CZ)
(74) Representative: Sturm, Christoph

(56) References cited:
- WO-A1-99/28722
- GB-A- 2 076 591
- US-A- 3 969 923
- US-A- 4 475 407

## Description

The present patent application relates to a fluid flow detection device and to an assembly comprising such a fluid flow detection device.

In fluid systems like potable water systems it is common practice that a check valve is positioned within a fluid pipe, wherein the check valve is closed when the pressure differential across the check valve is smaller than an opening threshold, and wherein the check valve is opened when the pressure differential across the check valve is greater than the opening threshold. Said opening threshold is also often called cracking pressure of the check valve.

It is desired to detect a fluid flow in a fluid pipe downstream of a check valve when the check valve is closed in order to determine a leakage within the fluid systems. For the time being is it not possible to provide such a leakage detection function is a simple, reliable and cost-effective manner.

Prior art is disclosed by US 3 969 923 A, GB 2 076 591 A, WO 99/28722 A1 and US 4 475 407 A.

Against this background, a novel fluid flow detection device according to claim 1 is provided.

The novel fluid flow detection device provides a first bypass across the check valve with a first piston being positioned within the first bypass; wherein the first piston is designed to become transferred between a first position and a second position when the pressure differential across the check valve is greater than a first threshold that is smaller than the opening threshold, and wherein the first piston closes the first bypass in the first position and the second position.

The novel fluid flow detection device further provides a second bypass across the check valve with a second piston being positioned within the second bypass, wherein the second piston is designed to become transferred between a first position and a second position when the pressure differential across the check valve is greater than a second threshold that is greater than the first threshold but smaller than the opening threshold, and wherein the second piston closes the second bypass in the first position but opens the same in the second position.

The novel fluid flow detection device further comprises a first sensor for detecting the position of the first piston a second sensor for detecting the position of the second piston.

The novel fluid flow detection device provides a leakage detection function being simple, reliable and cost-effective.

According to a preferred embodiment of the invention, when the sensors detect that the first piston is in the second position but that the second piston is in the first position, a relatively low leakage fluid flow in the fluid pipe downstream of the closed check valve is detected. When the sensors detect that the first piston and the second piston are both in the respective second position, a relatively high leakage fluid flow in the fluid pipe downstream of the closed check valve is detected. When the sensors detect that the first piston and the second piston are both in the respective first position, no leakage fluid flow in the fluid pipe downstream of the closed check valve is detected. This provides a leakage detection function being simple, reliable and cost-effective.

Preferably, the the first piston carries a first magnet being detectable by the first sensor; and the second piston carries a second magnet being detectable by the second sensor. This provides a leakage detection function being simple, reliable and cost-effective.

According to a preferred embodiment of the invention, the first threshold is in a range between 10% and 40% of opening threshold, and that the second threshold is in a range between 50% and 80% of opening threshold. This provides a leakage detection function being simple, reliable and cost-effective.

Preferred developments of the invention are provided by the dependent claims and the description which follows. Exemplary embodiments are explained in more detail on the basis of the drawing, in which:
- Figure 1: shows a side view of a fluid flow detection device together with a fluid pipe and a check valve being positioned within the fluid pipe;
- Figure 2: shows a top view the assembly of Fig. 1;
- Figure 3: shows a cross section through the assembly of Fig. 1 and 2 in a first status of the same;
- Figure 4: shows a cross section through the assembly of Fig. 1 and 2 in a second status of the same; and
- Figure 5: shows a cross section through the assembly of Fig. 1 and 2 in a third status of the same.

The present application relates to a fluid flow detection device and to an assembly comprising such a fluid flow detection device.

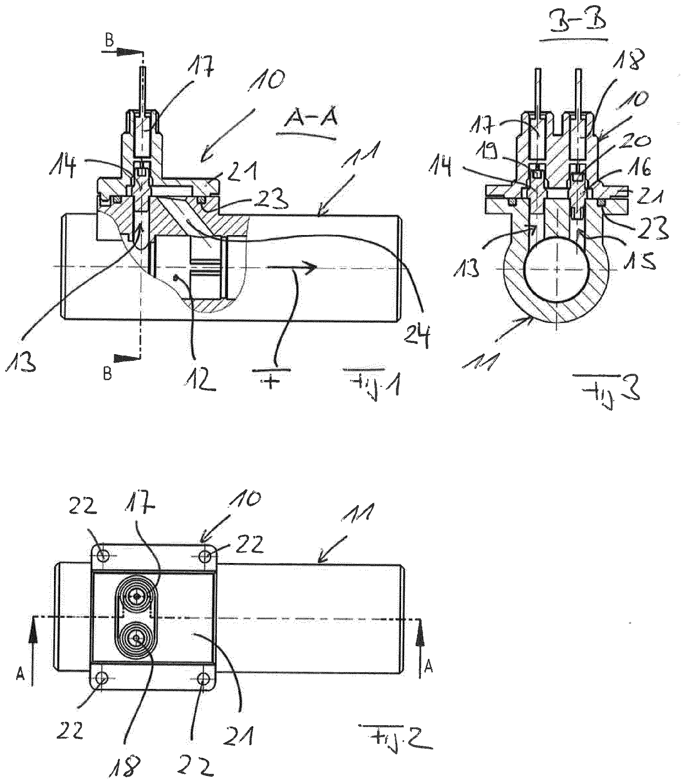

Figures 1 to 5 show details of fluid flow detection device 10 together with a fluid pipe 11 and a check valve 12 being positioned within the fluid pipe 11. Fluid flows through the fluid pipe 11 in flow direction F when the check valve 12 is opened.

The check valve 12 is closed when a pressure differential across the check valve 12 is smaller than an opening threshold. The opening threshold is also often called cracking pressure.

The check valve 12 is opened when the pressure differential across the check valve 12 is greater than the opening threshold.

The fluid flow detection device 10 serves to detect a fluid flow in a fluid pipe 11 downstream of a check valve 12 when the check valve 12 is closed. Such a fluid flow may occur from a leakage in the fluid system downstream of a check valve 12.

The fluid flow detection device 10 provides a first bypass 13 across the check valve 12 with a first piston 14 being positioned within the first bypass 13. The first piston 14 is designed to become transferred from a first position into a second position when the pressure differential across the check valve 12 is greater than a first threshold, wherein the first threshold is smaller than the opening threshold. The first piston 14 closes the first bypass 13 in the first position and the second position of the same. The first threshold is preferably in a range between 10% and 40% of opening threshold. E.g. the first threshold corresponds to 33% of opening threshold.

The fluid flow detection device 10 further provides a second bypass 15 across the check valve 12 with a second piston 16 being positioned within the second bypass 15. The second piston 16 is designed to become transferred from a first position into a second position when the pressure differential across the check valve 12 is greater than a second threshold, wherein said second threshold is greater than the first threshold but smaller than the opening threshold. The second piston 16 closes the second bypass 15 in the first position but opens the same in the second position. The second threshold is preferably in a range between 50% and 80% of opening threshold. E.g. the second threshold corresponds to 66% of opening threshold.

The fluid flow detection device 10 further comprises a first sensor 17 for detecting the position of the first piston 14 a second sensor 18 for detecting the position of the second piston 16.

The first piston 14 carries preferably a first magnet 19 being detectable by the first sensor 17. The second piston 16 carries preferably a second magnet 20 being detectable by the second sensor 18. The first sensor 17 and the second sensor 18 are preferably both provided by Hall sensors.

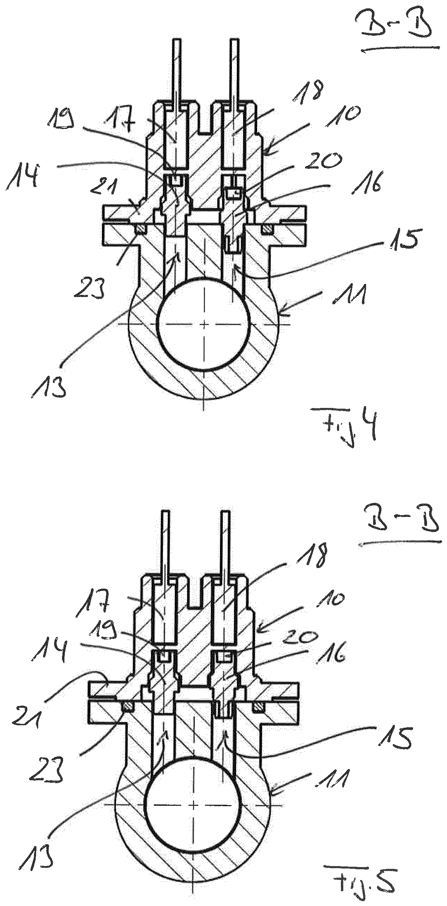

When the sensors 17, 18 detect that the first piston 14 is in the second position but that the second piston 16 is in the first position (see Fig. 4), a relatively low leakage fluid flow in the fluid pipe 11 downstream of the closed check valve 12 is detected.

When the sensors 17, 18 detect that the first piston 14 and the second piston 16 are both in the respective second position (see Fig. 5), a relatively high leakage fluid flow in the fluid pipe 11 downstream of the closed check valve 12 is detected.

When the sensors 17, 18 detect that the first piston 14 and the second piston 16 are both in the respective first position (see Fig. 3), no leakage fluid flow in the fluid pipe 11 downstream of the closed check valve 12 is detected.

The pistons 14, 16 and the sensors 17, 18 are accommodated within a common housing 21 of the fluid flow detection device 10. The housing 21 of the fluid flow detection device 10 is mounted to the fluid pipe 11 by screws 22, wherein a sealing element 23 is positioned between the housing 21 and the fluid pipe 11.

The bypasses 13, 15 are partially provided by the fluid pipe 11 and by the housing 21 of the fluid flow detection device 10. Downstream of the pistons 14, 16 the bypasses 13, 15 merge into a common section 24 of the same (see Fig. 1). The invention allows a simple and reliable leakage detection using low-cost parts and low-cost technology. The basis of the inventive concept is to detect the position of the pistons 14, 16. The pistons 14, 16 are preferably equipped with permanent magnets 19, 20. The movement of pistons 14, 16 is limited by stoppers at the first position and at the second position. The position of each piston 14, 16 is detected preferably by Hall sensor 17, 18.

The pistons 14, 16 are positioned in the bypasses 13, 15 around the spring operated check valve 12. When the fluid flow is high enough to reach differential pressure higher than cracking pressure of check valve 12, the bypass sensing by the fluid flow detection device 10 is without effect. When the check valve 12 is closed, the bypass sensing by the fluid flow detection device 10 is in effect.

As mentioned above, the first piston 14 closes the first bypass 13 in the first position and the second position of the same. The second piston 16 closes the second bypass 15 in the first position but opens the second bypass 15 in the second position. This is provided by a defined shape of the positions 14, 16.

Further on, the first piston 14 becomes transferred from the first position into the second position of the same when the pressure differential across the check valve 12 is greater than the first threshold, wherein the second piston 16 becomes transferred from the first position into the second position of the same when the pressure differential across the check valve 12 is greater than the second threshold. This is provided by a defined weight of pistons 14, 16 and magnets 17, 18 resulting in a defined gravitational force of the respective piston 14, 16, wherein the pressure differential needed to move the respective piston 14, 16 upwards from the first position into the second position of the same is a function of the cross section area of the respective piston 14, 16 and the weight of the same. The weight of the first piston 14 including the first magnet 19 is preferably smaller / lower than the weight of the second piston 16 including the second magnet 20.

When the first piston 14 is in the second position but that the second piston 16 is in the first position (see Fig. 4), the sensor 17 detects the second position of the first piston 14. In this status the fluid is unable to flow further, because the first piston 14 closes the first bypass 13 in all positions of first piston 14. When also the second piston 16 is in the second position (see Fig. 5), the sensor 18 detects the second position of the second piston 16. In this status the fluid is able to flow further, because the second piston 16 opens the second bypass 15 in the second position of the second piston 16.

When the first piston 14 has been transferred from his first position into his second position, and when further within specific time interval after that position change of the first piston 14 no position change is detected for the second piston 16 but the first piston 14 returns to his first position within said time interval, a relatively low leakage fluid flow in the fluid pipe 11 downstream of the check valve 12 is detected.

When the first piston 14 has been transferred from his first position into his second position, and when further within specific time interval after that position change of the first piston 14 also a position change is detected for the second piston 16, a relatively high leakage fluid flow in the fluid pipe 11 downstream of the check valve 12 is detected. The corresponding leakage amount can be calculated from the time period for which the second piston 16 stays in his second position.

The invention is preferably used in potable water systems.

### List of reference signs

- 10: fluid flow detection device
- 11: fluid pipe
- 12: check valve
- 13: bypass
- 14: piston
- 15: bypass
- 16: piston
- 17: sensor
- 18: sensor
- 19: magnet
- 20: magnet
- 21: housing
- 22: screw
- 23: sealing element
- 24: section

## Claims

1. Fluid flow detection device (10) for detecting a fluid flow in a fluid pipe (11) downstream of a spring operated check valve (12) being positioned within the fluid pipe (11) when the check valve (12) is closed, wherein the check valve (12) is closed when the pressure differential across the spring operated check valve (12) is smaller than an opening threshold, and wherein the check valve (12) is opened when the pressure differential across the spring operated check valve (12) is greater than the opening threshold
having a first piston (14) being positioned within a first bypass (13) across the check valve (12); wherein the first piston (14) is designed to become transferred from a first position into a second position when the pressure differential across the check valve (12) is greater than a first threshold that is smaller than the opening threshold,
**characterized by**
the first piston (14) having a shape that closes the first bypass (13) in the first position of the first piston (14) and the second position of the first piston (14);
a second piston (16) being positioned within a second bypass (15) across the check valve (12), wherein the second piston (16) is designed to become transferred from a first position into a second position when the pressure differential across the check valve (12) is greater than a second threshold that is greater than the first threshold but smaller than the opening threshold, and wherein the second piston (16) has a shape that closes the second bypass (15) in the first position of the second piston (16) but opens the same in the second position of the second piston (16);
a first sensor (17) for detecting the position of the first piston (14);
a second sensor (18) for detecting the position of the second piston (16);
a housing (21) that accommodates the pistons (14, 16) and the sensors (17, 18), wherein the bypasses (13, 15) are partially provided by the fluid pipe (11) and by the housing (21).

2. Fluid flow detection device as claimed in claim 1, **characterized in that**
the first piston (14) carries a first magnet (19) being detectable by the first sensor (17), wherein the first piston (14) and the first magnet (19) have a weight and the first piston (14) has a cross section area so that the first piston (14) becomes transferred from its first position into its second position when the pressure differential across the check valve (12) is greater than the first threshold;
the second piston (16) carries a second magnet (20) being detectable by the second sensor (18), wherein the second piston (16) and the second magnet (20) have a weight and the second piston (16) has a cross section area so that the second piston (16) becomes transferred from its first position into its second position when the pressure differential across the check valve (12) is greater than the second threshold.

3. Fluid flow detection device as claimed in claim 1 or 2, **characterized in that** the first sensor (17) and the second sensor (18) are both Hall sensors.

4. Fluid flow detection device as claimed in one claims 1 to 3, **characterized in that** it is so configured that
when the sensors (17, 18) detect that the first piston (14) is in the second position but that the second piston (16) is in the first position, a relatively low leakage fluid flow in the fluid pipe (11) downstream of the check valve (12) is detected;
when the sensors (17, 18) detect that the first piston (14) and the second piston (16) are both in the respective second position, a relatively high leakage fluid flow in the fluid pipe (11) downstream of the check valve (12) is detected;
when the sensors (17, 18) detect that the first piston (14) and the second piston (16) are both in the respective first position, no leakage fluid flow in the fluid pipe downstream of the check valve (12) is detected.

5. Fluid flow detection device as claimed in one of claims 1 to 4, **characterized in that** the first threshold is in a range between 10% and 40% of opening threshold, and that the second threshold is in a range between 50% and 80% of opening threshold.

6. Fluid flow detection device as claimed in claim 2 or 3, **characterized in that** the first threshold correspond to almost 33% of opening threshold, and that the second threshold correspond to almost 66% of opening threshold.

7. Assembly comprising
a fluid pipe (11),
a spring operated check valve (12) being positioned within the fluid pipe (11), wherein the spring operated check valve (12) is closed when the pressure differential across the check valve (12) is smaller than an opening threshold, and wherein the check valve (12) is opened when the pressure differential across the check valve (12) is greater than the opening threshold,
a flow detection device (10) adapted to detect a fluid flow in a fluid pipe (11) downstream of the spring operated check valve (12) when the spring operated check valve (12) is closed,
**characterized by**
said fluid flow detection device (10) being as claimed in one of claims 1 to 6.

## Patentansprüche

1. Fluidströmungsdetektionsvorrichtung (10) zum Detektieren einer Fluidströmung in einem Fluidrohr (11) stromabwärts eines federbetätigten Sperrventils (12), das innerhalb des Fluidrohrs (11) angeordnet ist, wenn das Sperrventil (12) geschlossen ist, wobei das Sperrventil (12) geschlossen ist, wenn der Druckunterschied über dem federbetätigten Sperrventil (12) kleiner ist als ein Öffnungsschwellenwert, und wobei das Sperrventil (12) geöffnet ist, wenn der Druckunterschied über dem federbetätigten Sperrventil (12) größer ist als der Öffnungsschwellenwert,
mit einem ersten Kolben (14), der innerhalb einer ersten Nebenleitung (13) über dem Sperrventil (12) angeordnet ist, wobei der erste Kolben (14) ausgelegt ist, um von einer ersten Position in eine zweite Position überführt zu werden, wenn der Druckunterschied über dem Sperrventil (12) größer ist als ein erster Schwellenwert, der kleiner ist als der Öffnungsschwellenwert,
**gekennzeichnet durch**
eine Form des ersten Kolbens (14), die die erste Nebenleitung (13) in der ersten Position des ersten Kolbens (14) und der zweiten Position des ersten Kolbens (14) verschließt,
einen zweiten Kolben (16), der innerhalb einer zweiten Nebenleitung (15) über dem Sperrventil (12) angeordnet ist, wobei der zweite Kolben (16) ausgelegt ist, um von einer ersten Position in eine zweite Position überführt zu werden, wenn der Druckunterschied über dem Sperrventil (12) größer ist als ein zweiter Schwellenwert, der größer als der erste Schwellenwert aber kleiner als der Öffnungsschwellenwert ist, und wobei der zweite Kolben (16) eine Form aufweist, die die zweite Nebenleitung (15) in der ersten Position des zweiten Kolbens (16) verschließt, aber dieselbe in der zweiten Position des zweiten Kolbens (16) öffnet;
einen ersten Sensor (17) zum Detektieren der Position des ersten Kolbens (14),
einen zweiten Sensor (18) zum Detektieren der Position des zweiten Kolbens (16),
ein Gehäuse (21), das die Kolben (14, 16) und die Sensoren (17, 18) beherbergt, wobei die Nebenleitungen (13, 15) teilweise durch das Fluidrohr (11) und durch das Gehäuse (21) bereitgestellt sind.

2. Fluidströmungsdetektionsvorrichtung nach Anspruch 1, **dadurch gekennzeichnet, dass**
der erste Kolben (14) einen ersten Magnet (19) trägt, der durch den ersten Sensor (17) detektierbar ist, wobei der erste Kolben (14) und der erste Magnet (19) ein Gewicht aufweisen und der erste Kolben (14) eine Querschnittsfläche aufweist, so dass der erste Kolben (14) von seiner ersten Position in seine zweite Position überführt wird, wenn der Druckunterschied über dem Sperrventil (12) größer ist als der erste Schwellenwert,
der zweite Kolben (16) einen zweiten Magnet (20) trägt, der durch den zweiten Sensor (18) detektierbar ist, wobei der zweite Kolben (16) und der zweite Magnet (20) ein Gewicht aufweisen und der zweite Kolben (16) eine Querschnittsfläche aufweist, so dass der zweite Kolben (16) von seiner ersten Position in seine zweite Position überführt wird, wenn der Druckunterschied über dem Sperrventil (12) größer ist als der zweite Schwellenwert.

3. Fluidströmungsdetektionsvorrichtung nach Anspruch 1 oder 2, **dadurch gekennzeichnet, dass** sowohl der erste Sensor (17) als auch der zweite Sensor (18) Hall-Sensoren sind.

4. Fluidströmungsdetektionsvorrichtung nach einem der Ansprüche 1 bis 3, **dadurch gekennzeichnet, dass** sie derart ausgelegt ist, dass
wenn die Sensoren (17, 18) detektieren, dass sich der erste Kolben (14) in der zweiten Position befindet, aber der zweite Kolben (16) sich in der ersten Position befindet, eine verhältnismäßig geringe Leckfluidströmung in dem Fluidrohr (11) stromabwärts des Sperrventils (12) detektiert wird,
wenn die Sensoren (17, 18) detektieren, dass sich sowohl der erste Kolben (14) als auch der zweite Kolben (16) in der jeweils zweiten Position befinden, eine verhältnismäßig hohe Leckfluidströmung in dem Fluidrohr (11) stromabwärts des Sperrventils (12) detektiert wird,
wenn die Sensoren (17, 18) detektieren, dass sich sowohl der erste Kolben (14) als auch der zweite Kolben (16) in der jeweils ersten Position befinden, keine Leckfluidströmung im Fluidrohr stromabwärts des Sperrventils (12) detektiert wird.

5. Fluidströmungsdetektionsvorrichtung nach einem der Ansprüche 1 bis 4, **dadurch gekennzeichnet, dass** der erste Schwellenwert in einem Bereich zwischen 10 % und 40 % des Öffnungsschwellenwerts liegt, und dass der zweite Schwellenwert in einem Bereich zwischen 50 % und 80 % des Öffnungsschwellenwerts liegt.

6. Fluidströmungsdetektionsvorrichtung nach Anspruch 2 oder 3, **dadurch gekennzeichnet, dass** der erste Schwellenwert fast 33 % des Öffnungsschwellenwerts entspricht, und dass der zweite Schwellenwert fast 66 % des Öffnungsschwellenwerts entspricht.

7. Anordnung, umfassend:
ein Fluidrohr (11),
ein federbetätigtes Sperrventil (12), das innerhalb des Fluidrohrs (11) angeordnet ist, wobei das federbetätigte Sperrventil (12) geschlossen ist, wenn der Druckunterschied über dem Sperrventil (12) kleiner ist als ein Öffnungsschwellenwert, und wobei das Sperrventil (12) geöffnet ist, wenn der Druckunterschied über dem Sperrventil (12) größer ist als der Öffnungsschwellenwert,
eine Strömungsdetektionsvorrichtung (10), die geeignet ist, um eine Fluidströmung in einem Fluidrohr (11) stromabwärts des federbetätigten Sperrventils (12) zu detektieren, wenn das federbetätigte Sperrventil (12) geschlossen ist,
**dadurch gekennzeichnet, dass**
die Fluidströmungsdetektionsvorrichtung (10) wie in einem der Ansprüche 1 bis 6 ist.

## Revendications

1. Dispositif de détection d'écoulement de fluide (10) destiné à détecter un écoulement de fluide dans un tuyau de fluide (11) en aval d'un clapet antiretour à ressort (12) qui est positionné à l'intérieur du tuyau de fluide (11) quand le clapet antiretour (12) est fermé, le clapet antiretour (12) étant fermé quand le différentiel de pression à travers le clapet antiretour à ressort (12) est inférieur à un seuil d'ouverture, et le clapet antiretour (12) étant ouvert quand le différentiel de pression à travers le clapet antiretour à ressort (12) est supérieur au seuil d'ouverture,
ayant un premier piston (14) qui est positionné à l'intérieur d'une première dérivation (13) d'un côté à l'autre du clapet antiretour (12) ; le premier piston (14) étant conçu pour être transféré d'une première position à une deuxième position quand le différentiel de pression à travers le clapet antiretour (12) est supérieur à un premier seuil qui est inférieur au seuil d'ouverture,
**caractérisé par** le premier piston (14) ayant une forme qui ferme la première dérivation (13) dans la première position du premier piston (14) et la deuxième position du premier piston (14) ;
un deuxième piston (16) qui est positionné à l'intérieur d'une deuxième dérivation (15) d'un côté à l'autre du clapet antiretour (12), le deuxième piston (16) étant conçu pour être transféré d'une première position à une deuxième position quand le différentiel de pression à travers le clapet antiretour (12) est supérieur à un deuxième seuil qui est supérieur au premier seuil, mais inférieur au seuil d'ouverture, et le deuxième piston (16) ayant une forme qui ferme la deuxième dérivation (15) dans la première position du deuxième piston (16), mais ouvre celle-ci dans la deuxième position du deuxième piston (16) ;
un premier capteur (17) pour détecter la position du premier piston (14) ;
un deuxième capteur (18) pour détecter la position du deuxième piston (16) ;
un boîtier (21) qui accueille les pistons (14, 16) et les capteurs (17, 18), les dérivations (13, 15) étant partiellement fournies par le tuyau de fluide (11) et par le boîtier (21).

2. Dispositif de détection d'écoulement de fluide selon la revendication 1, **caractérisé en ce que**
le premier piston (14) porte un premier aimant (19) qui est détectable par le premier capteur (17), le premier piston (14) et le premier aimant (19) ayant un poids et le premier piston (14) ayant une surface de section transversale telle que le premier piston (14) est transféré de sa première position à sa deuxième position quand le différentiel de pression à travers le clapet antiretour (12) est supérieur au premier seuil ;
le deuxième piston (16) porte un deuxième aimant (20) qui est détectable par le deuxième capteur (18), le deuxième piston (16) et le deuxième aimant (20) ayant un poids et le deuxième piston (16) ayant une surface de section transversale telle que le deuxième piston (16) est transféré de sa première position à sa deuxième position quand le différentiel de pression à travers le clapet antiretour (12) est supérieur au deuxième seuil.

3. Dispositif de détection d'écoulement de fluide selon la revendication 1 ou 2, **caractérisé en ce que** le premier capteur (17) et le deuxième capteur (18) sont tous deux des capteurs à effet Hall.

4. Dispositif de détection d'écoulement de fluide selon une des revendications 1 à 3, **caractérisé en ce qu'**il est configuré de telle sorte que
lorsque les capteurs (17, 18) détectent que le premier piston (14) est dans la deuxième position, mais que le deuxième piston (16) est dans la première position, un écoulement de fluide de fuite relativement faible dans le tuyau de fluide (11) en aval du clapet antiretour (12) est détecté ;
lorsque les capteurs (17, 18) détectent que le premier piston (14) et le deuxième piston (16) sont tous deux dans la deuxième position respective, un écoulement de fluide de fuite relativement fort dans le tuyau de fluide (11) en aval du clapet antiretour (12) est détecté ;
lorsque les capteurs (17, 18) détectent que le premier piston (14) et le deuxième piston (16) sont tous deux dans la première position respective, aucun écoulement de fluide de fuite dans le tuyau de fluide en aval du clapet antiretour (12) n'est détecté.

5. Dispositif de détection d'écoulement de fluide selon une des revendications 1 à 4, **caractérisé en ce que** le premier seuil se situe dans une gamme entre 10 % et 40 % du seuil d'ouverture, et **en ce que** le deuxième seuil se situe dans une gamme entre 50 % et 80 % du seuil d'ouverture.

6. Dispositif de détection d'écoulement de fluide selon la revendication 2 ou 3, **caractérisé en ce que** le premier seuil correspond à environ 33 % du seuil d'ouverture, et **en ce que** le deuxième seuil correspond à environ 66 % du seuil d'ouverture.

7. Ensemble comprenant
un tuyau de fluide (11),
un clapet antiretour à ressort (12) qui est positionné à l'intérieur du tuyau de fluide (11), le clapet antiretour à ressort (12) étant fermé quand le différentiel de pression à travers le clapet antiretour (12) est inférieur à un seuil d'ouverture, et le clapet antiretour (12) étant ouvert quand le différentiel de pression à travers le clapet antiretour (12) est supérieur au seuil d'ouverture,
un dispositif de détection de fluide (10) adapté pour détecter un écoulement de fluide dans un tuyau de fluide (11) en aval du clapet antiretour à ressort (12) quand le clapet antiretour à ressort (12) est fermé,
**caractérisé par**
ledit dispositif de détection de fluide (10) qui est selon une des revendications 1 à 6.
